# EUROPÄISCHE PATENTANMELDUNG

(11) **EP 4 650 145 A1**
(43) Veröffentlichungstag der Anmeldung: **19.11.2025**
(21) Anmeldenummer: 25172528.9
(22) Anmeldetag: 25.04.2025
(51) Int. Cl.: B29C 49/42, A61L 2/08, A61L 2/10, B29C 49/12, B29C 49/36, B29C 49/46, B29K 67/00, B29L 31/00, B65B 55/08

(54) **VERFAHREN UND VORRICHTUNG ZUR STERILISATION VON BEHÄLTERN**

(30) Priorität: 16.05.2024 DE 102024113740
(71) Anmelder: KHS GmbH, 44143 Dortmund (DE)
(72) Erfinder: Gerhards, Martin, 44143 Dortmund (DE); Herold, Thomas, 44143 Dortmund (DE)
(74) Vertreter: Eisenführ Speiser

(57) **Zusammenfassung**

Die vorliegende Erfindung betrifft ein Verfahren zum Umformen von Vorformlingen aus einem thermoplastischen Material zu fertigen Behältern in einer Streckblasmaschine (1), bei dem ein Vorformling (2) entlang eines Transportpfades durch Abschnitte der Streckblasmaschine (1) geführt wird, wobei das Verfahren die folgenden Schritte umfasst: Aufbringen eines Sterilisierfluides auf eine äußere Oberfläche eines Vorformlings (2) und Einbringen eines Sterilisierfluides in einen Innenraum des Vorformlings (2) vor einem Umformen des Vorformlings (1) in einer Streckblasstation (S5), Umformen der Vorformlinge (2) zu fertigen Behältern (3) in der Streckblasstation (S5) und anschließendes Sterilisieren von ersten Handhabungsbereichen (14, 15, 16, 21, 23, 24) der fertigen Behälter (3) in einer Kontaktflächensterilisiervorrichtung (S7), wobei die ersten Handhabungsbereiche (14, 15, 16, 21, 23, 24) der Behälter (3) bis zum Eintreffen der Behälter (3) bei der Kontaktflächensterilisiervorrichtung (S7) zum Transport der Behälter (3) eingerichtet sind und die Behälter (3) ab dem Eintreffen der Behälter (3) bei der Kontaktflächensterilisiervorrichtung (S7) mittels zweiter Handhabungsbereiche (22) transportiert werden, die von den ersten Handhabungsbereichen (21) verschieden sind. Die Erfindung betrifft ebenfalls eine entsprechende Vorrichtung.

## Beschreibung

Die vorliegende Erfindung betrifft ein Verfahren und eine Vorrichtung zum Umformen von Vorformlingen aus einem thermoplastischen Material zu fertigen, mindestens bereichsweise sterilen Behältern in einer Blasmaschine, insbesondere in einer Streckblasmaschine, bei denen Vorformlinge und Behälter entlang eines Transportpfades durch Abschnitte der Blas- bzw. Streckblasmaschine geführt werden.

Bekannt ist die Herstellung von Behältern durch Blasformen aus Vorformlingen aus einem thermoplastischen Material, beispielsweise aus Vorformlingen aus PET (Polyethylenterephthalat). Dabei werden die Vorformlinge innerhalb einer Blasformmaschine, hier auch als Vorrichtung zum Umformen von Vorformlingen bezeichnet, unterschiedlichen Bearbeitungsstationen zugeführt. Typischerweise weist eine Blasformmaschine eine Heizvorrichtung zum Temperieren bzw. zum thermischen Konditionieren der Vorformlinge sowie eine Blaseinrichtung mit wenigstens einer Blasstation auf, in deren Bereich der jeweils zuvor temperaturkonditionierte Vorformling zu einem Behälter expandiert wird. Im vorliegenden Kontext stehen Streckblasmaschinen im Vordergrund, bei denen der Vorformling mittels einer Reckstrange während des Expandierens gereckt wird. Möglichkeiten der Temperierung der Vorformlinge sind beispielsweise in der DE 23 52 926 A1 erläutert. Unter Temperierung oder thermischer Konditionierung wird dabei verstanden, dass der Vorformling auf eine für die Umformung geeignete Temperatur erwärmt und dem Vorformling ggf. ein Temperaturprofil in Längsrichtung und/oder in Umfangsrichtung aufgeprägt wird.

Die Expansion in den fertigen Behälter erfolgt beispielsweise mit Hilfe eines Druckgases, insbesondere Druckluft, als Druckmedium, das mit einem Formdruck in den zu expandierenden Vorformling eingeleitet wird. Der verfahrenstechnische Ablauf bei einer derartigen Expansion des Vorformlings wird in der DE 43 40 291 A1 erläutert. Der grundsätzliche Aufbau einer Blasstation ist in der DE 42 12 583 A1 beschrieben. Gemäß einem typischen Weiterverarbeitungsverfahren werden die durch Blasformen hergestellten Behälter einer nachfolgenden Fülleinrichtung zugeführt und hier mit dem vorgesehenen Produkt oder Füllgut gefüllt. Es ist zwar auch möglich, Behälter aus Vorformlingen herzustellen und dabei gleichzeitig mit einem Füllgut zu befüllen, welches als hydraulisches Druckmedium zum Expandieren des Vorformlings bzw. zum Ausformen des Behälters mit einem Form- und Fülldruck zugeführt wird. Diese Art eines Umformverfahrens und entsprechende Vorrichtungen zum Umformen von Vorformlingen sind von der vorliegenden Erfindung nicht umfasst.

Unter einem Sterilisieren (auch Sterilisation) wird vorliegend verstanden, dass z.B. unter Verwendung chemischer Sterilisierfluide eine keimtötende Behandlung erfolgt. Die vorliegende Offenbarung ist unter dem Aspekt zu sehen, dass z.B. keimempfindliche Getränke unter aseptischen bzw. sterilen Bedingungen abgefüllt werden müssen, um die gewünschte Haltbarkeit zu erreichen. Dies setzt z.B. voraus, dass die Behälter, in die die Getränke abgefüllt werden, diese Sterilbedingungen erfüllen, also mindestens auf den füllgutberührten Oberflächen weitgehend keimfrei sind, derart dass eine Sterilisierungsbehandlung durchgeführt wurde, um Mikroorganismen abzutöten. Zu diesem Zweck können fertige Behälter vor der Befüllung oder Vorformlinge vor ihrer Umformung in Behälter sterilisiert werden, wobei nachfolgend dann eine Neuverkeimung verhindert wird. So können sowohl die Vorformlinge als auch später die daraus hergestellten Behälter sterilisiert werden. Die Sterilisierung des Vorformlings hat gegenüber der Sterilisierung des daraus hergestellten Behälters den Vorteil, dass die zu sterilisierende Oberfläche viel kleiner ist, so dass die einzusetzende Menge an Sterilisierfluid geringer ausfallen kann.

Üblicherweise ist nach dem Blasformvorgang eine Spülung mit einem sterilen Spülfluid vorgesehen, um den fertigen Behälter von den Spuren des Sterilisierfluides zu befreien. Dies ist bspw. in der WO2014/139624A1 beschrieben. Der Blasformvorgang selbst führt bereits zu einem Spüleffekt, denn das verwendete Blasgas und das Ablassen des verwendeten Blasgases trägt zum Entfernen des Sterilisierfluides bei, soweit dieses noch vor der Umformung im Vorformling enthalten ist. Auch kann ein Sterilisierfluid, z.B. Wasserstoffperoxid, zerfallen und ist somit nicht mehr im Vorformling oder dem Behälter enthalten.

Aus der DE 10 2014 010 283 A1 ist auch bekannt, z.B. einen Vorformling mit einem Sterilisierfluid zu durchspülen, während der Vorformling bereits in der Umformstation aufgenommen ist. Ebenso ist es bekannt, einen Vorformling vor dem Erreichen der Umformstation einer Sterilisierung zu unterziehen (WO 2010/020530 A1), z.B. auf dem Weg zwischen der Heizvorrichtung und der Umformstation, oder z.B. vor dem Einlaufen des Vorformlings in die Heizvorrichtung einer Blasformmaschine, oder z.B. vor einem Einlauf eines Vorformlings in eine Blasformmaschine, z.B. im Bereich einer Zuführschiene für die Vorformlinge. Aus der EP 2 588 295 A1 ist auch bekannt, einen Vorformling innerhalb der Heizvorrichtung zu sterilisieren.

Eine bekannte Vorgehensweise bei der Sterilisation von Vorformlingen ist z.B., dass Wasserstoffperoxid (H₂O₂) in den Vorformling dosiert, der Vorformling zusammen mit dem dosierten Wasserstoffperoxid in der Heizvorrichtung erwärmt und im Anschluss ein fertiger Behälter geblasen wird. Dabei zerfällt der überwiegende Teil des eingesetzten Wasserstoffperoxids in Sauerstoff und Wasser. Bekannt ist aber auch, die Zudosierung von Wasserstoffperoxid zwischen der Heizvorrichtung und der Umformvorrichtung vorzusehen. Bei Entspannung auf Umgebungsdruck wird der fertige Behälter mit dem 25- bis 30-fachen des Flaschenvolumens gespült und damit etwaig verbliebenes Wasserstoffperoxid auf einen tolerierbaren Wert abgesenkt.

Aus der WO2015/024641 sind ein Verfahren und eine Vorrichtung zur Blasformung zumindest bereichsweise steriler Behälter bekannt. Dabei werden die Vorformlinge und Behälter über den gesamten Transportpfad in einem mit Sterilgas gefüllten Korridor geführt und dadurch kontinuierlich steril gehalten. Zusätzlich sind im Sterilgaskorridor sterilisierende Strahlungsquellen angeordnet die den Kanal und die Behälter, insbesondere im Mündungsbereich sterilisieren.

Die zuvor genannten Verfahren und Vorrichtungen haben alle noch ihre spezifischen Nachteile und sind teilweise aufwändig und kostenintensiv. Die vorliegende Erfindung hat sich zur Aufgabe gestellt, ein effizientes und sicheres Verfahren und eine ebensolche Vorrichtung bereitzustellen, welche eine effizientere Sterilisierung ermöglichen.

Die vorliegende Offenbarung stellt ein Verfahren zum Umformen von Vorformlingen bereit, bei dem aus einem thermoplastischen Material fertige, zumindest bereichsweise sterile Behälter hergestellt werden. Dieses Verfahren findet bevorzugt in einer Streckblasmaschine statt. Innerhalb des Umformverfahrens wird ein Vorformling entlang eines Transportpfades durch Abschnitte (oder Stationen, Sektionen) der Streckblasmaschine geführt.

In einem Behandlungsschritt (Hauptsterilisation) wird der Vorformling sterilisiert, indem Sterilisierfluid auf eine äußere Oberfläche des Vorformlings und/oder in einen Innenraum des Vorformlings eingebracht wird. Die Sterilisation kann zumindest teilweise oder vollständig vor, während oder nach einem Erwärmen des Vorformlings in einer Heizvorrichtung, und/oder vor oder während des Umformens innerhalb einer Streckblaseinrichtung (Blasstation, Blasrad) erfolgen. Die Heizvorrichtung kann in bekannter Art eingerichtet sein, um die Vorformlinge zu temperieren und so auf die richtige Temperatur für die Umformung zu bringen. Die Hauptsterilisation oder erste Sterilisation des Vorformlings erfolgt jedoch spätestens beim Umformen des Vorformlings in der Streckblasstation (Streckblasstation, Blasrad, Blasstation) der Streckblasmaschine. In der Streckblasstation werden die Vorformlinge zu fertigen Behältern umgeformt.

Gemäß einem Aspekt der vorliegenden Erfindung werden nach der Umformung und vor einem Befüllen und/oder Verschließen der Behälter an den Handhabungsbereichen der fertigen Behälter, die nachfolgend auch als erste Kontaktflächen bzw. erste Handhabungsbereiche bezeichnet werden, in einer Kontaktflächensterilisiervorrichtung sterilisiert. Diese ersten Kontaktflächen befinden sich in oder an den ersten Handhabungsbereichen auf der Oberfläche des Behälters oder auch der Vorformlinge. Daher werden die ersten Kontaktflächen auch als die Kontaktflächen der ersten Handhabungsbereiche bezeichnet. An oder um diese ersten Handhabungsbereiche herum greifen die unterschiedlichen Transporteinrichtungen und/oder Behandlungseinrichtungen vorzugsweise an den Behältern an und gelangen dadurch mit den Behältern an den ersten Kontaktflächen in Kontakt, bevor die Behälter zur Kontaktflächensterilisiervorrichtung gelangen. Vorteilhaft werden also in einem abschließenden Schritt die ersten Handhabungsbereiche an den Behältern gezielt und begrenzt sterilisiert.

Diese ersten Handhabungsbereiche sind bspw. an oder in der Umgebung von einem oder mehreren Neckringen, Nuten oder einem Gewinde der Behälter. Es können ein Neckring oder auch mehrere Neckringe an einem Behälter vorgesehen sein. Ein erster Handhabungsbereich kann auch unterhalb oder oberhalb eines oder mehrerer Neckringe oder innerhalb einer Nut sein. Es können auch Transportdorne in einen Behälter eingreifen. Daher kann ein erster Handhabungsbereich bzw. können die Kontaktflächen eines ersten Handhabungsbereichs auch innerhalb der Mündung eines Behälters liegen. Insgesamt befinden sich die Kontaktflächen der ersten Handhabungsbereiche vorzugsweise am bzw. im Mündungsbereich der Behälter. Die Kontaktflächen der ersten Handhabungsbereiche der Behälter sind stromaufwärts, also vor dem Eintreffen der Behälter an der Kontaktflächensterilisiervorrichtung mit einer oder mehreren Transport- und/oder Behandlungseinrichtungen in Kontakt gekommen. Im Vordergrund der vorliegenden Offenbarung steht die gezielte und weitgehend begrenzte Sterilisierung ersten Handhabungsbereiche in der Kontaktflächensterilisiervorrichtung.

Zudem werden die Behälter ab dem Eintreffen der Behälter bei der Kontaktflächensterilisiervorrichtung mittels zweiter Handhabungsbereiche transportiert, die von den ersten Handhabungsbereichen verschieden sind. Dadurch wird gewährleistet, dass die sterilisierten ersten Handhabungsbereiche nicht mehr kontaktiert und dadurch rekontaminiert werden. In anderen Worten werden die Behälter beim Sterilisieren und nach dem Sterilisieren der (ersten Kontaktflächen) ersten Handhabungsbereiche der Behälter innerhalb der Kontaktflächensterilisiervorrichtung nur noch außerhalb der sterilisierten ersten Handhabungsbereiche berührt, zumindest bis die Behälter verschlossen bzw. befüllt und verschlossen sind.

Gemäß einem weiteren Aspekt liegen die ersten Handhabungsbereiche näher an einem Mündungsbereich der Behälter als die zweiten Handhabungsbereiche. Insbesondere können die ersten Handhabungsbereiche am Mündungsbereich der Behälter liegen und diese ggf. mindestens teilweise bilden. Der besonders kritische Mündungsbereich der Behälter wird dadurch weiter vor einer Neukontaminierung geschützt.

Vorteilhaft können die Behälter innerhalb der Kontaktflächensterilisiervorrichtung mit einer Transfervorrichtung transportiert werden, welche die Behälter mittels geeigneter Transporteinrichtungen nur außerhalb der ersten Handhabungsbereiche der Behälter berührt. Mit anderen Worten kann die Kontaktflächensterilisiervorrichtung so eingerichtet sein, dass die Behälter nur noch an (zweiten Kontaktflächen) zweiten Handhabungsbereich der Behälter berührt werden, wobei die zweiten Handhabungsbereiche bzw. zweiten Kontaktflächen von den ersten Handhabungsbereichen bzw. zweiten Kontaktflächen verschieden sind.

Die Transporteinrichtung der Transfervorrichtung der Kontaktflächensterilisiervorrichtung kann vorteilhaft so ausgestaltet sein, dass sie die Behälter im Bereich des Schafts und der Kuppel kontaktiert und nicht am oder im Mündungsbereich. Die zweiten Kontaktflächen bzw. zweiten Handhabungsbereiche befinden sich somit vorteilhaft im Bereich eines Schafts und/oder einer Kuppel der Behälter.

Die Transporteinrichtung der Transfervorrichtung der Kontaktflächensterilisiervorrichtung kann vorteilhaft als Transporttasche, Transportband oder auch als Greifer ausgestaltet sein, die jeweils so ausgestaltet sind, dass sie die Behälter nur noch an den zweiten Kontaktflächen bzw. zweiten Handhabungsbereichen kontaktieren. Die Sterilisierung der ersten Kontaktflächen bzw. der ersten Handhabungsbereiche in der Kontaktflächensterilisiervorrichtung kann auf unterschiedliche Arten erfolgen. Grundsätzlich können auch sterilisierende Fluide oder Aerosole eingesetzt werden. Besonders vorteilhaft ist jedoch eine Sterilisierung durch Bestrahlen der ersten Handhabungsbereiche bzw. ersten Kontaktflächen mit einer sterilisierenden Strahlungsquelle.

Diese sterilisierende Strahlungsquelle kann jede Strahlungsquelle sein, die eine Strahlung mit sterilisierender Wirkung emittiert. Vorteilhaft wird UV-Strahlung bzw. UV-C-Strahlung eingesetzt, wobei die Strahlungsquelle zumindest anteilig ausreichende Strahlung im UV-Bereich oder UV-C-Bereich abstrahlen sollte. Ultraviolettstrahlung, kurz UV, UV-Strahlung, UV-Licht, ist elektromagnetische Strahlung im optischen Frequenzbereich (Licht) mit kürzeren Wellenlängen als das für den Menschen sichtbare Licht. Allgemein werden drei Bereiche der UV-Strahlung unterschieden: UV-A im Bereich von 380 nm bis 315 nm, UV-B im Bereich von 315 nm bis 280 nm und UV-C im Bereich von 280 bis 100 nm. Die UV-Strahlung im Kontext der vorliegenden Offenbarung liegt mit Vorteil in einem Wellenlängenbereich von 280 nm bis 200 nm. Es kommen grundsätzlich aber auch energieärmere Wellenlängenbereiche der UV-Strahlung in Betracht, wobei deren Wirkung dann entsprechend geringer ausfallen kann.

Eine für die vorliegende Anwendung geeigneten sterilisierende Strahlungsquelle kann bspw. eine Pulslichtquelle (gepulste Lichtquelle) sein, bei der mittels Hochspannungsimpulsen im kV-Bereich (bspw. 10 kV bis 60 kV) ein Gas, vorteilhaft Xenon, ionisiert wird, so dass sich ein Lichtbogen bzw. Lichtblitz bildet. Die Dauer des Lichtblitzes kann kleiner als 1 Millisekunde, vorteilhaft im Bereich von 100 µs bis 900 µs und noch vorteilhafter 300 µs betragen. Die Strahlungsintensität liegt vorteilhaft im Bereich von einigen kW/cm² und auch vorteilhaft bei 1 kW/ cm². Die Strahlungsintensität eines Blitzes kann bei diesen Strahlungsquellen ein Zehntausendfaches der Intensität der Sonnenstrahlung auf der Erde entsprechen. Das abgestrahlte Spektrum einer solchen Strahlungsquelle kann dem weißen Lichts entsprechen und kann im Bereich von 200 nm bis 11000 nm liegen, also den UV-Bereich (insbesondere den UV-C-Bereich), den sichtbaren Bereich und den Infrarotbereich abdecken. Dabei kann der Anteil an UV-Strahlung vorteilhaft größer als 5 %, auch vorteilhaft größer als 10 % und mit Vorteil größer als oder gleich 15 % des gesamten abgestrahlten Spektrums sein. Innerhalb des Spektrums der Strahlung dieser Art von gepulsten Lichtquellen ist ausreichend Energie innerhalb des UV-Bereichs, insbesondere innerhalb des UV-C-Bereichs vorhanden, um Mikroorganismen in sehr kurzer Bestrahlungszeit abzutöten. So können bspw. nur ein Puls, zwei oder drei Pulse (Lichtblitze) ausreichen, um die Sterilisation durchzuführen.

Eine weitere vorteilhafte sterilisierende Strahlungsquelle kann eine Elektronenstrahlquelle (E-Beam) sein. Bei dieser sogenannte "Elektronenstrahlsterilisation" (kurz: E-Beam) besteht der Strahl aus einem konzentrierten, stark aufgeladenen Elektronenstrom und wird von Beschleunigern als Impuls- oder Dauerstrahl erzeugt. Wenn der Elektronenstrahl auf den zu sterilisierenden Bereich gerichtet wird, wird Energie von den Elektronen absorbiert, wodurch diverse chemische Verbindungen verändert werden. Insbesondere werden dadurch auch DNA und Mikroorganismen zerstört. Der Elektronenstrahl wird mittels Hochenergie-Elektronenbeschleunigern erzeugt. Der E-Beam ist also eine Form von ionisierter Energie, die sich durch niedrige Penetration und hohe Dosisleistung auszeichnet. Der E-Beam kann aufgrund der kurzen Expositionsdauer für bestimmte Materialien (bspw. Polypropylen) vorteilhaft sein.

Als sterilisierende Strahlungsquellen kommen auch Nieder-, Mittel- oder Hochdruckdampflampen, wie bspw. Quecksilberdampflampen in Betracht. Die Lampen können je nach Anforderungen schwerpunktmäßig im UV-A-Bereich um 400 nm und bis in den UV-C-Bereich um 250 nm emittieren.

Mit Vorteil kommen auch LEDs (Light-Emitting Diodes) als sterilisierende Strahlungsquellen in Betracht, die eine ausreichende Strahlung im UV-Bereich, vorteilhaft im UV-C-Bereich haben.

Mit Vorteil werden die die Behälter von der Kontaktflächensterilisiervorrichtung bzw. von der Transporteinrichtung der Transfervorrichtung der Kontaktflächensterilisiervorrichtung, bevorzugt unmittelbar, in einen Teil-Reinraum oder Reinraum überführt, wobei der Reinraum mindestens die sterilisierten ersten Kontaktflächen der ersten Handhabungsbereiche umschließt.

Der Vorteil der hier erläuterten Aspekte und Merkmale besteht darin, dass die kontinuierliche Aufrechterhaltung der Sterilität oder Keimarmut an der Mündung bzw. Außenseite der Vorformlinge und/oder Behälter nicht mehr über den gesamten Transportpfad erforderlich ist. Durch die nachgeschaltete Sterilisation der ersten Kontaktflächen bzw. ersten Handhabungsbereiche im Bereich der Mündung der Behälter und die nachfolgende Vermeidung des Kontakts mit den sterilisierten Flächen unmittelbar bevor die Behälter in einen Reinraum übergeben werden, ist sichergestellt, dass der Reinraum nicht kontaminiert wird und eine auf der Innenseite und zumindest im Mündungsbereich innen und außen sterile bzw. keimarme Behälter an eine Fülleinheit übergeben wird, ohne dass diese im Füller nachsterilisiert werden muss.

Die vorliegende Offenbarung stellt auch eine Vorrichtung, insbesondere eine Streckblasmaschine bereit, die allgemein zum Umformen von Vorformlingen aus einem thermoplastischen Material zu fertigen, zumindest bereichsweise sterilen Behältern eingerichtet ist. In der Vorrichtung wird ein Vorformling entlang eines Transportpfades stromabwärts durch Abschnitte der Vorrichtung bzw. Streckblasmaschine geführt wird. Die Vorrichtung umfasst mindestens eine oder mehrere Hauptsterilisationseinrichtungen, eine Heizvorrichtung zum Temperieren der Vorformlinge, eine Streckblasstation, eine Kontaktflächensterilisiervorrichtung, und eine Fülleinheit (Füller) zum Befüllen der fertigen Behälter mit einem Füllgut. Die genannten Stationen der Vorrichtung können jeweils stromabwärts zueinander entlang eines Transportpfades angeordnet sind, wobei die Hauptsterilisationseinrichtung(en) auch nach der Heizeinrichtung oder innerhalb der Streckblasstation vorgesehen sein kann/können. Die Hauptsterilisationseinrichtung ist vorteilhaft konfiguriert, um ein Sterilisierfluid auf eine äußere Oberfläche eines Vorformlings oder Behälters und/oder in einen Innenraum des Vorformlings oder Behälters einzubringen um dadurch die äußere und/oder innere Oberfläche der Vorformlinge und/oder Behälter zu sterilisieren. Die Streckblasstation ist eingerichtet, um die Vorformlinge zu fertigen Behältern umzuformen.

Die Kontaktflächensterilisationsvorrichtung kann ausgestaltet sein, um erste Kontaktflächen bzw. erste Handhabungsbereiche auf der äußeren Oberfläche der fertigen Behälter zu sterilisieren.

Vorteilhaft können zur Sterilisierung in der Kontaktflächensterilisiervorrichtung eine oder mehrere sterilisierende Strahlungsquellen vorgesehen sein, die auf die entsprechenden ersten Kontaktflächen bzw. ersten Handhabungsbereiche gerichtet sind. Die ersten Kontaktflächen bzw. ersten Handhabungsbereiche der Behälter können stromaufwärts mit einer oder mehreren Transport- und/oder Behandlungseinrichtungen, insbesondere im Mündungsbereich, in Kontakt gekommen sein, wobei die Behälter beim und nach dem Sterilisieren der ersten Kontaktflächen bzw. ersten Handhabungsbereiche ohne weiteren Kontakt an den ersten Kontaktflächen bzw. ersten Handhabungsbereichen derart von der Kontaktflächensterilisiervorrichtung transportiert werden, dass die Behälter nur außerhalb der sterilisierten ersten Handhabungsbereiche (bzw. ersten Kontaktflächen) berührt werden. Von der Kontaktflächensterilisiervorrichtung können die Behälter unmittelbar in einen (Teil-)Reinraum überführt, wobei der Reinraum mindestens den Bereich der sterilisierten Kontaktflächen umschließt.

Durch die genannten Aspekte und Merkmals werden die Anforderungen an die Sterilisierungseinrichtungen der Streckblasmaschine reduziert. Im Extremfall ist nur noch eine Sterilisationseinrichtung bzw. die Sterilität bzw. Keimarmut erhaltende Vorrichtung erforderlich, nämlich die Kontaktflächensterilisiervorrichtung. Es muss nicht mehr an jeder kritischen Übergabestelle nach der Hauptsterilisation eine zusätzliche Sterilisation durchgeführt werden.

Die Kontaktflächensterilisiervorrichtung kann eine Transfervorrichtung und eine Sterilisationseinrichtung umfassen. Mit anderen Worten kann für die Kontaktflächensterilisiervorrichtung eine geeignete Transfervorrichtung um eine geeignete Sterilisationseinrichtung ergänzt werden.

Um die erforderliche Behandlungszeit auf einer möglichst kurzen Strecke zu realisieren, kann die Transfervorrichtung mit der Sterilisationseinrichtung linear ausgeführt werden. Wenn in der Fülleinheit ein Reinraum nur im Bereich der Mündung definiert ist, kann ein Behälter an die Fülleinheit übergeben werden, der nur innen und im Bereich der Mündung außen steril ist. Dadurch können weiter Maßnahmen zur Sterilisation der Vorform- bzw. Behälteraußenwand ausgenommen der Mündungsbereich, entfallen.

Mit anderen Worten werden gemäß der vorliegenden Offenbarung die Behälter nach der Entnahme der Behälter aus der Umformeinrichtung (Streckblasstation) mittelbar oder unmittelbar (also mit oder ohne weitere dazwischenliegende Transfervorrichtungen) an eine Transfervorrichtung übergeben, auf der eine Sterilisationseinrichtung dermaßen angeordnet ist, dass die ersten Handhabungsbereiche im Bereich der Mündung der Behälter außen und innen sterilisiert werden. Idealerweise erfolgt die Sterilisation mit UV-Strahlung (vorteilhaft UV-C-Strahlung). Dazu können mit Vorteil Pulslichtquellen (bspw. Gasentladungslampen) oder Elektronenstrahlquellen, oder auch LEDs mit einer Emission im UV-C-Bereich eingesetzt werden. Vorteilhaft können die Sterilisationseinrichtung und die Transfervorrichtung der Kontaktflächensterilisiervorrichtung mindestens teilweise linear ausgeführt werden, um das Verhältnis Verweilzeit und Streckenlänge der Sterilisationseinrichtung zu optimieren. Die Transporteinrichtung der Transfervorrichtung in der Kontaktflächensterilisiervorrichtung hat keinen Kontakt zur zu sterilisierenden Mündung. Sie kann beispielsweise als Saugtasche auf dem Flaschenkörper, als Transportband oder auch als Greifelement ausgeführt werden.

Mit Vorteil kann die Transfervorrichtung so ausgestaltet sein, dass ihre Transporteinrichtungen in einem Bereich, in dem sie ohne Behälter unterwegs ist, durch chemische Verfahren, thermische Verfahren und/oder Strahlung steril gehalten werden. Das unterstützt dabei, eine Neuverkeimung der Flaschenaußenseite durch die Transporteinrichtung zu vermeiden.

In einer Ausgestaltung können die sterilisierenden Strahlungsquellen auch beidseitig der Behälter angeordnet sein. Dadurch kann bspw. die Bestrahlungsdauer und/oder Bestrahlungsintensität reduziert werden.

Die einzelnen sterilisierenden Strahlungsquellen können gezielt im Abstand zu den ersten Handhabungsbereichen bzw. ersten Kontaktflächen der Behälter eingestellt werden, z.B. zur Anpassung an sich ändernde Behältergeometrien.

Vorteilhaft kann mindestens eine sterilisierende Strahlungsquelle der Sterilisiereinrichtung der Kontaktflächensterilisiervorrichtung zentral von oben auf die Mündungsöffnung der Behälter gerichtet sein.

Die Mehrzahl an sterilisierenden Strahlungsquellen können in bzw. an einem gemeinsamen Träger angeordnet sein. Entscheidend ist die Anordnung der sterilisierenden Strahlungsquellen und deren Ausrichtung auf die ersten Kontaktflächen bzw. ersten Handhabungsbereiche.

Vorteilhaft können die sterilisierenden Strahlungsquellen abgeschirmt sein. Das Abschirmen der Strahlung kann erfolgen, indem eine abschirmende Umhausung vorgesehen ist, die sich wenigstens entlang des Bereiches des Transportpfades erstreckt, in dem die Strahlungsquellen angeordnet sind.

Die gesamte Einstrahlzeit der sterilisierenden Strahlung, insbesondere UV-C-Strahlung, kann in der Größenordnung von kleiner als 5 Sekunden, bevorzugt im Bereich 1 Sekunde bis 2 Sekunden liegen. Weiter vorteilhaft kann die Bestrahlungsdauer bzw. Einstrahlzeit auch im Bereich von 0,5 Sekunden bis 1 Sekunde liegen. Insbesondere bei Pulslichtquellen kann die Einstrahlzeit noch kürzer sein.

Vorteilhaft kann die Strahlungsquelle mehrteilig ausgestaltet und derart eingerichtet sein, dass die ersten Kontaktflächen bzw. ersten Handhabungsbereiche am Mündungsabschnitt des Behälters gezielt und möglichst begrenzt angestrahlt werden. Weiter vorteilhaft können der oder die Strahlungsquellen abstandsveränderlich in Bezug die ersten Kontaktflächen bzw. die ersten Handhabungsflächen des Behälters angeordnet sein.

Nachfolgend wird die Offenbarung anhand von bevorzugten Ausführungsbeispielen und anhand der beiliegenden Figuren näher erläutert. Die Zeichnungen sind nicht unbedingt maßstabsgetreu. In den Figuren sind gleiche oder im Wesentlichen funktionsgleiche bzw. -ähnliche Elemente üblicherweise mit den gleichen Bezugszeichen bezeichnet. Es zeigen:
- Fig. 1: eine vereinfachte schematische Draufsicht einer Vorrichtung zum Umformen von Vorformlingen am Beispiel einer Streckblasmaschine gemäß einem Ausführungsbeispiel,
- Fig. 2: eine vereinfachte schematische Draufsicht einer Vorrichtung zum Umformen von Vorformlingen am Beispiel einer Streckblasmaschine gemäß einem weiteren Ausführungsbeispiel,
- Fig. 3: eine vereinfachte schematische seitliche Darstellung eines Behälters zur Illustrierung der ersten und zweiten Kontaktflächen bzw. ersten und zweiten Handhabungsbereiche und
- Fig. 4: eine vereinfachte schematische seitliche Darstellung eines Behälters beim Durchlaufen der Kontaktflächensterilisiereinrichtung.

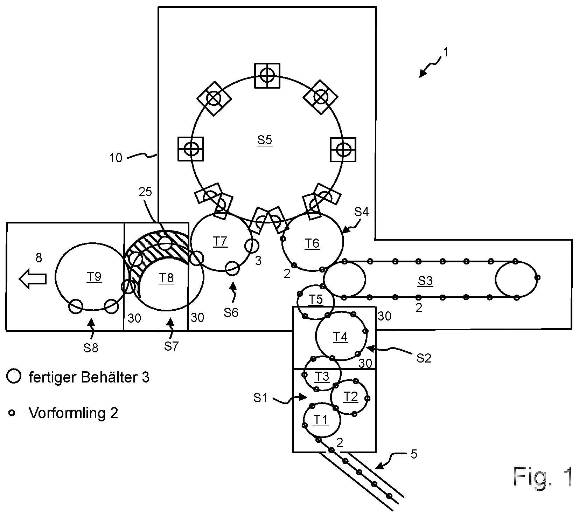

Fig. 1 ist eine vereinfachte schematische Draufsicht einer Vorrichtung zum Umformen von Vorformlingen 2 am Beispiel einer Streckblasmaschine 1, die aus Vorformlingen 2 fertige Behälter 3 herstellt. Die Streckblasmaschine 1 ist von einem Maschinengehäuse 10 umgeben und umfasst mehrere Abschnitte oder auch Sektionen. Beispielhaft sind hier die Sektionen S1, S2, S3, S4, S5, S6, S7 und S8 dargestellt. In anderen Ausführungen können es weniger oder auch mehr Sektionen sein. Innerhalb der Sektionen gibt es Transfervorrichtungen (z.B. Transferräder) T1 bis T9 und Behandlungsvorrichtungen in Form einer Streckblasstation S5 und einer Heizvorrichtung S3 zum Erwärmen der Vorformlinge 2.

Entlang eines Transportpfades erfolgt der Transport der Vorformlinge 2 und Behälter 3 in der folgenden Weise stromabwärts. Im Rahmen einer Zuführung 5, z.B. ausgebildet als Zuführschiene oder als Luftförderer, durchlaufen die Vorformlinge 2 zunächst die erste Sektion S1, durch die sie mittels der Transfervorrichtungen T1, T2 und T3 (Transferräder) gefördert werden.

Die zweite Sektion S2 ist nach der ersten Sektion S1 angeordnet und wird von den Vorformlingen 2 im Wesentlichen mittels der Transfervorrichtung T4 durchlaufen. Dort werden die Vorformlinge 2 im Rahmen einer Hauptsterilisation mit Sterilisierfluid beaufschlagt. Das Sterilisierfluid wird auf die Vorformlinge 2 in bekannter Weise von innen und außen aufgebracht. Dabei kann das Sterilisierfluid dampf- oder aerosolförmig zugeführt werden, wobei sich die Vorformling 2 während dieses Zuführens auf einer Temperatur befinden, die unterhalb der Kondensationstemperatur des dampf- oder aerosolförmigen Sterilisierfluides liegt, sodass sich das Sterilisierfluid auf den Oberflächen der Vorformlinge 2 als Kondensat niederschlägt. Dabei erfolgt die Dampfzuführung so, dass sich ein weitgehend vollständiger Kondensationsfilm auf der Außen- und ggf. Innenseite des Vorformlings 2 ausbildet. Im weiteren Verlauf wird das Sterilisierfluid wieder von den Vorformlingen 2 entfernt bzw. kann sich gemäß entsprechender Reaktionsgleichungen zersetzen.

Im Anschluss an die vierte Sektion S4 (T4) und der Beaufschlagung mit Sterilisierfluid werden die Vorformlinge 2 an die Transfervorrichtung T5 übergeben und gelangen von dort zur dritten Sektion S3, in der sich die Heizvorrichtung befindet. Dort werden die Vorformlinge 2 in bekannter Weise mittels Heizeinrichtungen (bspw. Heizstrahler) erwärmt (temperiert). Von der Heizvorrichtung S3 gelangen die Vorformlinge 2 über die vierte Sektion S4 und die in dieser Sektion angeordnete Transfervorrichtung T6 zu einer Streckblasstation (auch Blasrad oder Blasstation) S5. In der Streckblasstation S5 werden die temperierten Vorformlinge 2 in bekannter Weise zu fertigen Behältern 3 geformt. Die fertigen Behälter 3 werden in der sechsten Sektion S6 an die Transfervorrichtung T7 übergeben. Da die Vorformlinge 2 und Behälter 3 nach der Hauptsterilisation in Sektion 2 mit diversen Transporteinrichtungen und Behandlungseinrichtungen in Kontakt gekommen sind, kann eine Neuverkeimung, insbesondere des sensiblen Mündungsbereiches der Behälter nicht ausgeschlossen werden.

Gemäß der vorliegenden Offenbarung ist nun eine siebte Sektion S7 vorgesehen, in der sich eine Kontaktflächensterilisiervorrichtung S7 befindet. Diese Kontaktflächensterilisiervorrichtung S7 besteht in diesem Ausführungsbeispiel im Wesentlichen aus einer Transfervorrichtung T8 (hier als ein Transferrad angedeutet) und einer Sterilisiereinrichtung 25. Von der Kontaktflächensterilisiereinrichtung S7 gelangen die Behälter 3 weiter zur achten Sektion S8, die als Teil-Reinraum oder Reinraum ausgestaltet ist und eine Transfervorrichtung T9 aufweist. In der achten Sektion 8 befindet sich eine Fülleinheit (Füller) zum Befüllen der Behälter mit einem Füllgut. Dort werden die Behälter 3 unter sterilen Reinraumbedingungen befüllt und verschlossen und anschließend, wie mit dem Pfeil 8 angedeutet, weitergeleitet. Die Kontaktflächensterilisiereinheit S7 befindet sich also nach der Streckblasstation S5 und unmittelbar vor der achten Sektion S8, die einen Teil-Reinraum oder Reinraum aufweist.

Wo erforderlich sind Zwischenwände 30 zur Abgrenzung von Sektionen vorgesehen. Hierzu gehören vor allem die achte Sektion S8, die als Reinraum ausgestaltet ist und die zweite Sektion S2 in der das Sterilisierfluid im Rahmen der Hauptsterilisation auf die Vorformlinge 2 aufgebracht wird.

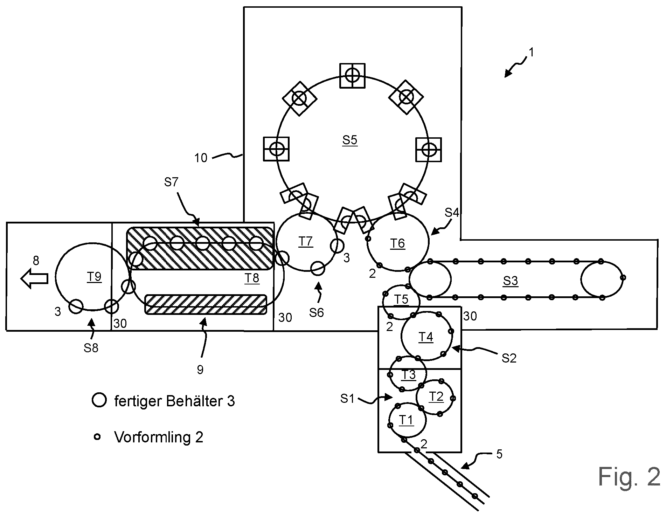

Fig. 2 ist eine vereinfachte schematische Draufsicht einer Vorrichtung zum Umformen 1 von Vorformlingen am Beispiel einer Streckblasmaschine 1 gemäß einem weiteren Ausführungsbeispiel. Grundsätzlich sind die Sektionen S1 bis S8 im Wesentlichen gleich wie bezüglich des Ausführungsbeispiels gemäß Figur 1 beschrieben. In diesem Ausführungsbeispiel ist die Kontaktflächensterilisiereinheit S7 mit einer linearen Transfervorrichtung T8 ausgestattet. Die Wegstrecke der Behälter 3 entlang des Transportpfades durch die Sterilisationseinrichtung 25 wird dadurch verlängert. Die Dauer der Sterilisation (Bestrahlung) kann dadurch besser eingestellt werden. Zudem ist eine weitere Sterilisationseinheit 9 vorgesehen, welche die Transporteinrichtungen der Transfervorrichtung T8 sterilisiert während diese zurücklaufen. Das verbessert die Sterilität der Behälter 3 weiter.

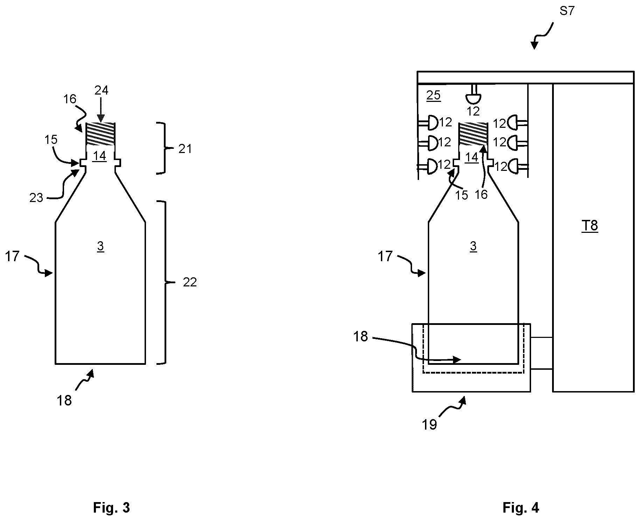

Fig. 3 ist eine vereinfachte schematische seitliche Darstellung eines Behälters 3 zur Illustrierung der ersten Kontaktflächen 21 und zweiten Kontaktflächen 22 bzw. ersten Handhabungsbereiche 21 und zweiten Handhabungsbereiche 22. Der Behälter 3 weist einen Mündungsbereich 14, einen Schaft 17 und einen Boden 18 auf. Im Mündungsbereich 14 befindet sich beispielhaft ein Neckring 15 und ein Gewinde 16. Der Behälter 3 ist natürlich von oben offen. Durch den Pfeil 24 ist somit ein innerer Bereich bzw. eine Öffnung des Mündungsbereichs 14 angedeutet. Im gesamten Mündungsbereich 14 (innen und außen) befinden sich die ersten Kontaktflächen 21 bzw. ersten Handhabungsbereiche 21, die mittels der Kontaktflächensterilisiereinrichtung S7 sterilisiert werden. Beispielsweise könnten Greifer (Transporteinrichtungen) von Transfervorrichtungen (bspw. T8) oberhalb und unterhalb (23) des Neckrings 15 angreifen. Dann befinden sich dort auch die ersten Handhabungsbereiche 21 bzw. ersten Kontaktflächen, die es zu sterilisieren gilt.

Fig. 4 ist eine vereinfachte schematische seitliche Darstellung eines Behälters 3 beim Durchlaufen der Kontaktflächensterilisiervorrichtung S7. Die Kontaktflächensterilisiereinheit S7 umfasst grundsätzlich eine Transfervorrichtung T8 und eine Sterilisiereinrichtung 25.

Die Sterilisierung der ersten Kontaktflächen bzw. der ersten Handhabungsbereiche 21 in der Kontaktflächensterilisiervorrichtung S7 kann auf unterschiedliche Arten erfolgen. Grundsätzlich können auch sterilisierende Fluide oder Aerosole eingesetzt werden. Besonders vorteilhaft ist jedoch eine Sterilisierung durch Bestrahlen der ersten Handhabungsbereiche 21 bzw. ersten Kontaktflächen 21 mit einer sterilisierenden Strahlungsquelle.

Diese sterilisierende Strahlungsquelle 12 kann jede Strahlungsquelle sein, die eine sterilisierende Wirkung hat. Vorteilhaft wird UV-Strahlung bzw. UV-C-Strahlung eingesetzt, wobei die Strahlungsquelle zumindest anteilig ausreichende Strahlung im UV-Bereich oder UV-C-Bereich abstrahlen sollte. Ultraviolettstrahlung, kurz UV, UV-Strahlung, UV-Licht, ist elektromagnetische Strahlung im optischen Frequenzbereich (Licht) mit kürzeren Wellenlängen als das für den Menschen sichtbare Licht. Allgemein werden drei Bereich der UV-Strahlung unterschieden: UV-A im Bereich von 380 nm bis 315 nm, UV-B im Bereich von 315 nm bis 280 nm und UV-C im Bereich von 280 bis 100 nm. Die UV-Strahlung im Kontext der vorliegenden Offenbarung liegt mit Vorteil in einem Wellenlängenbereich von 280 nm bis 200 nm. Es kommen grundsätzlich aber auch längere Wellenlängenbereiche der UV-Strahlung in Betracht, wobei deren Wirkung dann entsprechend geringer ausfallen kann.

Eine geeigneten sterilisierende Strahlungsquelle 12 kann bspw. eine Pulslichtquelle (gepulste Lichtquelle) sein, bei der mittels Hochspannungsimpulsen im kV-Bereich (bspw. 10 kV bis 60 kV) ein Gas, vorteilhaft Xenon, ionisiert wird, so dass sich ein Lichtbogen bzw. Lichtblitz bildet. Die Dauer des Lichtblitzes kann kleiner als 1 Millisekunde, vorteilhaft im Bereich von 100 µs bis 900 µs und noch vorteilhaft 300 µs betragen. Die Strahlungsintensität liegt vorteilhaft im Bereich von einigen kW/cm² und auch vorteilhaft 1 kW/ cm². Die Strahlungsintensität eines Blitzes kann ein Zehntausendfaches der Intensität der Sonnenstrahlung auf der Erde entsprechen. Das abgestrahlte Spektrum einer solchen Strahlungsquelle kann dem weißen Lichts entsprechen und kann im Bereich von 200 nm bis 11000 nm liegen, also den UV-Bereich (insbesondere den UV-C-Bereich), den sichtbaren Bereich und den Infrarotbereich abdecken. Dabei kann der Anteil an UV-Strahlung vorteilhaft größer als 5 %, auch vorteilhaft größer als 10 % und mit Vorteil größer als oder gleich 15 % des gesamten abgestrahlten Spektrums sein. In einer möglichen Ausgestaltung können eine oder mehrere Pulslichtquellen 12 oberhalb eines Behälters 3 angeordnet sein.

Eine weitere vorteilhafte sterilisierende Strahlungsquelle 12 kann eine Elektronenstrahlquelle (E-Beam) sein. Bei dieser sogenannte "Elektronenstrahlsterilisation" (kurz: E-Beam) besteht der Strahl aus einem konzentrierten, stark aufgeladenen Elektronenstrom und wird von Beschleunigern als Impuls- oder Dauerstrahl erzeugt. Wenn der Elektronenstrahl auf den zu sterilisierenden Bereich, also die ersten Handhabungsbereiche 21 gerichtet wird, werden Mikroorganismen zerstört. Der E-Beam kann aufgrund der kurzen Expositionsdauer für bestimmte Materialien (bspw. Polypropylen) vorteilhaft sein.

Als sterilisierende Strahlungsquellen 12 kommen auch Nieder- Mittel- oder Hochdruckdampflampen, wie bspw. Quecksilberdampflampen in Betracht. Die Lampen können je nach Anforderungen schwerpunktmäßig im UV-A-Bereich um 400 nm und bis in den UV-C-Bereich um 250 nm emittieren.

Es kommen auch LEDs (Light Emitting Diode) als sterilisierende Strahlungsquellen 12 in Betracht, die eine ausreichende Strahlung im UV-Bereich, vorteilhaft im UV-C-Bereich haben.

Diese Sterilisiereinrichtung 25 umfasst also allgemein eine oder mehrere weiter oben beschriebenen sterilisierende Strahlungsquellen 12, welche gezielt die ersten Kontaktflächen bzw. ersten Handhabungsbereiche 21 sterilisieren. Dazu sind diese Strahlungsquellen 12 so angeordnet, dass der äußere und innere Mündungsbereich 14 des Behälters 3 mit sterilisierende Strahlung beaufschlagt werden. Der Behälter 3 wird von der Transfervorrichtung T8 mittels der Transporteinrichtung 19 transportiert. Diese Transporteinrichtung 19 ist so ausgestaltet, dass der Behälter 3 nur innerhalb der zweiten Handhabungsbereiche 22 bzw. zweiten Kontaktflächen 22 kontaktiert wird. Die Transporteinrichtung 19 kann beispielsweise als Saugtasche, Transportband oder Greifer ausgestaltet sein, die jeweils nur im Bereich des Schafts 17 und/oder der Boden 18 der Behälter 3 angreifen.

### Bezugszeichenliste

- 1: Vorrichtung zum Umformen, Streckblasmaschine
- 2: Vorformling
- 3: fertiger Behälter
- 5: Zuführung, Zuführschiene für Vorformlinge
- 6: Mündungsabschnitt des Vorformlings
- 8: Richtung der Weiterbehandlung der Behälter
- 9: Sterilisiereinrichtung für Transporteinrichtungen von T8
- 10: Maschinengehäuse
- 14: Mündungsbereich des Behälters 3 (bspw. Flasche)
- 15: Neckring
- 16: Gewinde
- 17: Schaft des Behälters 3
- 18: Boden des Behälter 3
- 19: Transporteinrichtung 19
- 21: erste Kontaktflächen, erste Handhabungsbereiche
- 22: zweite Kontaktflächen, zweite Handhabungsbereiche
- 23: Bereich unterhalb des Neckrings
- 24: Mündungsöffnung des Behälters 3
- 25: Sterilisiereinrichtung der Kontaktflächensterilisiervorrichtung S7
- 30: Trennwände zwischen Sektionen
- S1: erste Sektion, Zuführung der Vorformlinge
- S2: zweite Sektion, Hauptsterilisation, Sterilisationseinrichtung
- S3: Dritte Sektion = Heizvorrichtung
- S4: vierte Sektion = Zuführvorrichtung, Zuführrad T6
- S5: fünfte Sektion = Streckblaseinrichtung, Streckblasstation, Blasrad
- S6: sechste Sektion = Abführvorrichtung, Abführrad T7
- S7: siebte Sektion = Kontaktflächensterilisiervorrichtung mit T8 und optional 9
- S8: achte Sektion = Reinraum mit Fülleinheit und T9
- T1: erste Transfervorrichtung, Transferrad, Vereinzelungsrad
- T2: zweite Transfervorrichtung, Transferrad
- T3: dritte Transfervorrichtung, Transferrad
- T4: vierte Transfervorrichtung, Transferrad, Bestandteil der Hauptsterilisation, Sterilisiereinrichtung
- T5: fünfte Transfervorrichtung, Transferrad
- T6: sechste Transfervorrichtung, Transferrad, Zuführrad
- T7: siebte Transfervorrichtung, Transferrad, Abführrad
- T8: achte Transfervorrichtung, Transferrad; Bestandteil der Kontaktflächensterilisiereinrichtung
- T9: neunte Transfervorrichtung, Transferrad, Fülleinheit, Füller

## Patentansprüche

1. Verfahren zum Umformen von Vorformlingen aus einem thermoplastischen Material zu fertigen Behältern in einer Blasmaschine, insbesondere Streckblasmaschine (1), bei dem Vorformlinge (2) und Behälter (3) entlang eines Transportpfades durch Abschnitte der Streckblasmaschine (1) geführt werden, wobei das Verfahren die folgenden Schritte umfasst: Aufbringen eines Sterilisierfluides auf eine äußere Oberfläche eines Vorformlings (2) und Einbringen eines Sterilisierfluides in einen Innenraum des Vorformlings (2) vor einem Umformen des Vorformlings (1) in einer Streckblasstation (S5), Umformen der Vorformlinge (2) zu fertigen Behältern (3) in der Streckblasstation (S5) und anschließendes Sterilisieren von ersten Handhabungsbereichen (14, 15, 16, 21, 23, 24) der fertigen Behälter (3) in einer Kontaktflächensterilisiervorrichtung (S7), wobei die ersten Handhabungsbereiche (14, 15, 16, 21, 23, 24) der Behälter (3) bis zum Eintreffen der Behälter (3) bei der Kontaktflächensterilisiervorrichtung (S7) zum Transport der Behälter (3) eingerichtet sind und die Behälter (3) ab dem Eintreffen der Behälter (3) bei der Kontaktflächensterilisiervorrichtung (S7) mittels zweiter Handhabungsbereiche (22) transportiert werden, die von den ersten Handhabungsbereichen (21) verschieden sind.

2. Verfahren nach Anspruch 1, wobei die ersten Handhabungsbereiche (21) näher an einem Mündungsbereich (14) der Behälter (3) angeordnet sind als die zweiten Handhabungsbereiche (22).

3. Verfahren nach Anspruch 1 oder 2, wobei die Behälter (3) von der Kontaktflächensterilisiervorrichtung (S7) (unmittelbar) in einen (Teil-)Reinraum (S8) überführt, wobei der (Teil-)Reinraum (S8) mindestens die sterilisierten ersten Handhabungsbereiche (21) umschließt.

4. Verfahren nach einem der Ansprüche 1 bis 3, wobei die ersten Handhabungsbereiche (21) in der Kontaktflächensterilisiervorrichtung (S7) mittels einer sterilisierenden Strahlungsquelle (12) sterilisierte werden.

5. Verfahren nach Anspruch 4, wobei die sterilisierende Strahlungsquelle (12) eine pulsierende Strahlungsquelle, eine UV-C-Strahlungsquelle und/oder einen E-Beam umfassen.

6. Verfahren nach einem der vorstehenden Ansprüche, wobei die ersten Handhabungsbereiche (21) in einem Mündungsbereich (14) der Behälter (3) angeordnet sind und einen Neckring (15), ein Gewinde (16) und/oder eine MündungsÖffnung (24) umfassen.

7. Vorrichtung, insbesondere Streckblasmaschine (1), zum Umformen von Vorformlingen (2) aus einem thermoplastischen Material zu fertigen zumindest bereichsweise sterilen Behältern (3), in die Vorformlinge (2) und die Behälter (3) entlang eines Transportpfades stromabwärts durch Abschnitte der Streckblasmaschine (1) geführt werden, wobei die Vorrichtung umfasst: eine Sterilisationseinrichtung (S2), eine Streckblasstation (S5) und eine Kontaktflächensterilisiervorrichtung (S7), die jeweils stromabwärts zueinander entlang des Transportpfades angeordnet sind, wobei die Sterilisationseinrichtung (S2) eingerichtet ist, um ein Sterilisierfluid auf eine äußere Oberfläche eines Vorformlings (2) und/oder Behälters (3) und in einen Innenraum (1) des Vorformlings (2) und/oder Behälters (3) einzubringen, die Streckblasstation (S5) eingerichtet ist, um die Vorformlinge (2) zu fertigen Behältern (3) umzuformen und die Kontaktflächensterilisationsvorrichtung (S7) eingerichtet ist, um erste Handhabungsbereiche (21) der Behälter (3) zu sterilisieren, wobei die Kontaktflächensterilisiervorrichtung (S7) eine Transfervorrichtung (T8) umfasst, die Transporteinrichtungen (19) aufweist, die eingerichtet sind, um die Behälter (3) an zweiten Handhabungsbereichen (22) zu kontaktieren und zu transportieren, wobei die zweiten Handhabungsbereiche (22) von der ersten Handhabungsbereichen (21) verschieden sind.

8. Vorrichtung nach Anspruch 7, wobei nur außerhalb der sterilisierten Kontaktflächen berührt werden und die Behälter (3) von der Kontaktflächensterilisiervorrichtung unmittelbar in einen (Teil-)Reinraum überführt, wobei der Reinraum mindestens den Bereich der sterilisierten Kontaktflächen umschließt.

9. Vorrichtung nach Anspruch 7 oder 8, wobei die Kontaktflächensterilisiervorrichtung (S7) eine Sterilisiereinrichtung (25) umfasst, welche sterilisierende Strahlungsquellen (12) aufweist, die eingerichtet sind, um die ersten Handhabungsbereiche (21) mit sterilisierender Strahlung zu beaufschlagen.
